Europäisches Patentamt

(19) European Patent Office

Office européen des brevets

(11) Veröffentlichungsnummer : **0 137 127**
**B1**

(12) **EUROPÄISCHE PATENTSCHRIFT**

(45) Veröffentlichungstag der Patentschrift :
09.09.87

(51) Int. Cl.⁴ : **C 07 D405/06**, A 61 K 31/41

(21) Anmeldenummer : **84107759.7**

(22) Anmeldetag : **04.07.84**

(54) **Verfahren zur Addition von Säuren an Glycidylverbindungen.**

(30) Priorität : 25.08.83 DE 3330640

(43) Veröffentlichungstag der Anmeldung :
17.04.85 Patentblatt 85/16

(45) Bekanntmachung des Hinweises auf die Patenterteilung : 09.09.87 Patentblatt 87/37

(84) Benannte Vertragsstaaten :
DE FR GB

(56) Entgegenhaltungen :
EP-A- 0 056 962
EP-A- 0 093 959
DE-A- 3 037 094
Die Akte enthält technische Angaben, die nach dem Eingang der Anmeldung eingereicht wurden und die nicht in dieser Patentschrift enthalten sind.

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Möller, Hinrich, Dr.**
**Schumannstrasse 11**
**D-4019 Monheim (DE)**

**Beschreibung**

Die Erfindung liegt auf dem Gebiet definierter niedermolekularer mehrfunktioneller N-Glycidyl-Verbindungen. Sie betrifft ein Verfahren zur Ringöffnung dieser Substanzen mit Halogen-Wasserstoffsäuren, Pseudohalogen-Wasserstoffsäuren und/oder Säuren des Phosphors unter zuverlässigem Erhalt von zumindest einer Epoxidgruppe.

Es ist bereits bekannt, mehrfunktionelle Glycidyl-Verbindungen, also z. B. Heterocyclen mit 3 oder mehr N-Glycidyl-Gruppen mit Protonensäuren so umzusetzen, daß nur eine Glycidyl-Gruppe der Ringöffnung unterzogen wird. Die deutsche Offenlegungsschrift 30 37 094 schlägt hierzu ein Verfahren vor, bei dem die mehrfunktionelle N-Glycidyl-Verbindung zunächst mit einem Überschuß an Säure behandelt wird und die Reaktion dann nach kurzer Zeit durch geeignete Maßnahmen, z. B. Neutralisation abgebrochen wird. In der europäischen Patentanmeldung EP-A-56 962 wird beschrieben, diese Vorgehensweise zur Derivatisierung von Triglycidylurazol einzusetzen. Nach diesen Verfahren werden jedoch nur Mischungen erhalten, die aus den Ausgangsverbindungen sowie Verbindungen, bei denen eine, zwei oder noch mehr Glycidyl-Gruppen ringgeöffnet sind, bestehen. Aus diesen Mischungen können nur mit Hilfe aufwendiger Trennoperationen, etwa mit Hilfe der Säulenchromatographie oder der Hochdruckflüssigkeits-Chromatographie reine Produkte gewonnen werden wie sie z. B. für den Einsatz als Arzneimittel gebraucht werden. Weiterhin neigen Glycidyl-Verbindungen in Gegenwart von starken Säuren zur Polymerisation. Es besteht daher Bedarf nach einem selektiven Ringöffnungsverfahren für Epoxide, das es erlaubt, unter Erhalt von mindestens einer Epoxidgruppe eine oder mehrere Epoxidgruppen mehrfunktioneller N-Glycidyl-Verbindungen in hohen Ausbeuten der Ringöffnung zu unterziehen.

Aufgabe der Erfindung ist somit die Schaffung eines derartigen Verfahrens. Eine weitere Aufgabe der Erfindung ist es, neue Derivate des Triglycidyl-Urazols bereitzustellen, welche zwei Glycidyl-Gruppen und eine Halogen-hydroxy-propylgruppe aufweisen.

Gegenstand der Erfindung ist somit in einer ersten Ausgestaltungsform ein Verfahren zur Herstellung definierter partieller Umsetzungsprodukte mehrfunktioneller N-Glycidylverbindungen mit Halogenwasserstoffsäuren, Pseudohalogenwasserstoffsäuren oder Säuren des Phosphors (unter Addition der Säuren an nur einen Teil der insgesamt vorliegenden Epoxidgruppen) dadurch gekennzeichnet, daß die mehrfunktionellen N-Glycidylverbindungen mit den Säuren und ihren Alkalimetallsalzen in wäßrigem Medium bei Temperaturen zwischen 0 und 60 °C im pH-Bereich zwischen 2 und 10 und dabei innerhalb einer Bandbreite von höchstens 2 Einheiten der pH-Skala bei einem Ansatzverhältnis von 1-20 mol Säure und ihrem Alkalimetallsalz pro mol umzusetzender Epoxidgruppe zur Reaktion gebracht werden, bis die vorbestimmte Menge an Epoxidgruppen abreagiert ist.

Das erfindungsgemäße Verfahren erreicht seine überraschend hohe Selektivität durch genaue Einhaltung von definierten Reaktionsbedingungen. So findet die Umsetzung in wäßrigem Milieu in einem Temperaturbereich zwischen 0 und 60 °C statt. Bevorzugt ist die Umsetzung bei Temperaturen zwischen 20 und 40 °C, insbesondere wird bei Raumtemperatur gearbeitet.

Für das erfindungsgemäße Verfahren ist die Konstanthaltung des pH-Wertes innerhalb einer Bandbreite von höchstens 2 pH-Einheiten von besonderer Wichtigkeit. Dabei kann die Reaktion bei einem pH-Wert zwischen 2 und 10, bevorzugt in einem pH-Bereich zwischen 3 und 8 und insbesondere zwischen 4 und 7 durchgeführt werden. Der gewünschte pH-Wert wird eingestellt, in dem man die gewünschte Menge eines Alkalimetallsalzes der zur Ringöffnung verwendeten Säure und erforderlichenfalls zusätzlich die erforderliche Menge der Säure selbst zugibt.

Nach einer weiteren Ausgestaltung der Erfindung ist es auch möglich, das Alkalimetallsalz, dessen Anion die Ringöffnung bewirken soll, vorzulegen und mit einer Fremdsäure, deren Anion unter Reaktionsbedingungen vergleichsweise schwer zur Addition an Glycidylverbindungen neigt, z. B. mit Schwefelsäure oder einer organischen Sulfonsäure, den pH-Wert konstant zu halten. Zur Konstanthaltung des pH-Werts während der gesamten Reaktionszeit wird dann Säure in dem Maße zum Reaktionsgemisch zudosiert, wie diese durch die fortschreitende Reaktion verbraucht werden. Die erfindungsgemäße Vorgehensweise bietet gleichzeitig den Vorteil, daß über den Säureverbrauch auch das Fortschreiten der Reaktion überwacht werden kann, das heißt man kann die Reaktion abbrechen, wenn z. B. genau 1 mol Säure pro mol mehrfunktionelle Glycidylverbindung verbraucht worden ist, oder beispielsweise wenn genau 2 mol Säure pro mol einer trifunktionellen Glycidylverbindung verbraucht worden sind. Im ersten Fall entstehen das Mono-Ringöffnungsprodukt in hoher Ausbeute, im zweiten Fall entstehen die Di-Ringöffnungsprodukte. Zum Abbrechen der Reaktion kann alkalisiert werden, gekühlt werden oder es können die Reaktionsprodukte mit Hilfe nicht wassermischbarer Lösungsmittel extrahiert werden. Bei Laboransätzen im Gramm-Maßstab wurden Reaktionszeiten zwischen 1 und 2 h zur Herstellung von mono-ringgeöffneten Verbindungen und 2 bis 3 h zur Herstellung von di-ringgeöffneten Produkten beobachtet.

Erfindungsgemäß werden die N-Glycidyl-Verbindungen mit Säuren und/oder ihren Alkalimetallsalzen umgesetzt. Geeignete Alkalimetallsalze sind die Natrium-, Kalium- oder Lithium-Salze. Pro mol umgesetzter Epoxidgruppe werden 1 bis 20 mol Säure und/oder Salz eingesetzt. Das Verhältnis der Alkalimetallsalze zu den freien Säuren richtet sich nach dem gewünschten pH-Wert. Der Überschuß an Säure

und/oder Salz ist von der Reaktivität des Anions abhängig. So ist bei reaktiven Anionen z. B. bei Jodanion nur ein kleiner überschuß oder aber das Arbeiten in molaren Mengen angezeigt, wohingegen bei Chlorid bevorzugt mit einem etwa 20-fachen Überschuß gearbeitet wird.

Eine besonders wichtige Ausführungsform des erfindungsgemäßen Verfahrens betrifft die Umsetzung der N-Glycidyl-Verbindungen mit Halogen-Wasserstoffsäuren und/oder den Alkalimetallhalogeniden. Danach ist es bevorzugt, die Umsetzung mit Mischungen aus Chlorwasserstoff/Natriumchlorid, Bromwasserstoff/Natriumbromid oder Kaliumbromid oder Jodwasserstoff/Kaliumjodid durchzuführen. Eine weitere Ausführungsform betrifft die Addition von Pseudo-Halogenwasserstoffsäuren. Unter Pseudo-Halogenwasserstoffsäuren sind z. B. die Blausäure, die Cyansäure und die Rhodanwasserstoffsäure zu verstehen. Besonders bevorzugt ist die Umsetzung mit ihren Natrium- oder Kaliumsalzen wie beispielsweise Kaliumcyanid, Natriumcyanat oder Natriumrhodanid. Dabei ist es bevorzugt, den pH-Wert durch kontinuierliche Zugabe von z. B. Schwefelsäure konstant zu halten. Eine weitere Ausführungsform der Erfindung betrifft die Addition von Säuren des Phosphors. Geeignete Säuren des Phosphors sind neben der Phosphorsäure und der phosphorigen Säure auch Phosphonsäuren mit organischem Rest z. B. Hydroxyethylendiphosphonsäure, Aminoethylendiphosphonsäure und andere mono- oder difunktionelle organische Phosphonsäuren. Bei den Säuren des Phosphors ist die Umsetzung mit sauren Salzen und/oder deren Mischungen bevorzugt. Saure Salze von Säuren des Phosphors sind Verbindungen, bei denen mindestens ein azider Wasserstoff nicht durch ein Metallkation z. B. ein Alkalimetallkation ersetzt worden ist. Auch hier wird der pH-Wert durch Zugabe der entsprechenden Säure konstant gehalten.

Erfindungsgemäß können zahlreiche N-Glycidyl-Verbindungen als Ausgangsmaterialien eingesetzt werden. Bevorzugt sind solche N-Glycidyl-Verbindungen, die durch Umsetzung einer N—H-aziden Verbindung mit einem Epihalogenhydrin hergestellt worden sind. Bevorzugte Ausgangsmaterialien im Sinne der Erfindung sind cyclische mehrfunktionelle N-Glycidyl-Verbindungen, die mehr als eine —NR—CO-Gruppe im Ring aufweisen. Unter diesen Verbindungen sind die Fünfring- und die Sechsring-Verbindungen sowie Verbindungen mit mehreren fünf und/oder sechs Ringen bevorzugt. Typische Vertreter der bevorzugten Ausgangsverbindungen sind mehrfunktionelle N-Glycidyl-Verbindungen auf Basis Urazol, Glykoluril, Hydantoin, Isocyanursäure, Barbitursäure, Harnsäure oder Purin.

Die mehrfunktionellen N-Glycidyl-Verbindungen dieser Heterocyclen werden erfindungsgemäß in Gegenwart von Wasser mit den Protonensäuren und/oder ihren Salzen umgesetzt. In den Fällen, in denen die Löslichkeit des Ausgangsproduktes zu gering ist, um die Reaktion durchführen zu können, also z. B. unter 1-g/100 g Wasser liegt, können lösungsvermittelnde Zusätze mit verwendet werden. Geeignete lösungsvermittelnde Zusätze sind unter Reaktionsbedingungen inerte Lösungsmittel, Solubilisatoren oder Tenside. So können Alkohole wie Methanol, Ethanol und Isopropanol, Ketone wie Aceton oder Amide wie Dimethylformamid als Lösungsmittel eingesetzt werden.

Die Aufarbeitung der Reaktionsprodukte kann nach unterschiedlichen Verfahren durchgeführt werden. So ist es beispielsweise möglich, die Reaktionsansätze der Gefriertrocknung zu unterwerfen und die Reaktionsprodukte mit organischen Lösungsmitteln z. B. Ethern oder chlorierten Kohlenwasserstoffen zu extrahieren. Es ist jedoch auch möglich, die flüssigen Reaktionsgemische mit Lösungsmitteln, die mit Wasser nicht mischbar sind, zu extrahieren. Geeignete Lösungsmittel sind Chloroform, Methylenchlorid, Diethylether oder auch Methyl-tertiär-Butylether. Eine weitere Reinigung der Reaktionsprodukte kann nach üblichen Verfahren der organischen Chemie, also z. B. durch Umkristallisation erfolgen.

Auch bei der Herstellung von Monoringöffnungsprodukten mehrfunktioneller N-Glycidyl-Verbindungen sind die Verfahrensprodukte vielfach chemisch nicht einheitlich, sondern stellen Isomerengemischen dar. So können erfindungsgemäß Produkte entstehen, die das als Substituent eintretende Säureanion in Dreistellung und die Hydroxylgruppe in Zweistellung des Propylrestes aufweisen. Weiterhin können jedoch auch die 2-3-Isomere entstehen. In den Fällen, in denen die Ausgangsverbindungen nicht drei gleiche N-Glycidyl-Gruppen tragen, entstehen weiterhin Isomere, bei denen unterschiedliche N-Glycidyl-Gruppen ringgeöffnet sind. In jedem Falle werden aber nahezu ausschließlich N-Glycidyl-Verbindungen mit der gewünschten Anzahl geöffneter Epoxidringe erhalten. Eine weitere Auftrennung der einzelnen Isomeren kann, wenn gewünscht, nach bekannten Methoden der organischen Chemie, etwa nach chromatographischen Methoden erfolgen.

Das erfindungsgemäße Verfahren ist besonders geeignet zur Herstellung von Derivaten des Triglycidyl-urazols. So können die Monoadditionsprodukte dieser Verbindung mit Chlorwasserstoff, Bromwasserstoff oder Jodwasserstoff in Ausbeuten von mehr als 50 %, beispielsweise mit einer Ausbeute von 79 % der Theorie nach dem erfindungsgemäßen Verfahren hergestellt werden.

Zur Herstellung von Additionsprodukten von Fluorwasserstoff an mehrfunktionelle Glycidylverbindungen stellt man zweckmäßigerweise zunächst in der beschriebenen Art ein anderes Halogenderivat etwa das Umsetzungsprodukt mit Chlorwasserstoff her und unterwirft dieses einer Substitutionsreaktion, bei der das andere Halogen durch Fluor ausgetauscht wird. So können beispielsweise Umsetzungsprodukte von 1,2,4-Triglycidylurazol mit Fluorwasserstoff erhalten werden, indem diese Verbindung unter Erhalt zweier Epoxidgruppen wie beschrieben mit Natriumchlorid/Chlorwasserstoff umgesetzt wird und anschließend Chlor gegen Fluor ausgetauscht wird.

Die Halogenaustauschreaktion wird bevorzugt in aprotischen dipolaren Lösungsmitteln wie Acetonitril, Aceton, Butanon, Dimethylformamid, Dimethylsulfoxid, Hexamethylphosphorsäuretriamid, N-Methylpyrrolidon-2-on, N-Formyl-piperidin oder in 1,2-Dimethoxyethan durchgeführt. Es ist jedoch auch möglich, in protischen Lösungsmitteln wie niederen Alkoholen ($C_2$-$C_4$ oder in Wasser oder in Mischungen der genannten Lösungsmittel zu arbeiten. Die Reaktionstemperatur wird zweckmäßigerweise zwischen 0 und 100 °C gewählt, bevorzugt läßt man bei 40 bis 80 °C reagieren. Im einzelnen sei hier auf Lehrbücher der präparativen organischen Chemie hingewiesen. Nach dem genannten Austauschverfahren kann Diglycidyl-(3-fluor-2-hydroxy-propyl)-urazol in guten Ausbeuten aus der entsprechenden Chlorverbindung hergestellt werden.

Nach dem erfindungsgemäßen Verfahren kann weiterhin Diglycidyl-(3-chlor-2-hydroxy-propyl)-isocyanurat hergestellt werden. Die Verbindung wird mit einer Ausbeute von 70 % d. Th. erhalten und läßt sich auch in das entsprechende Fluorderivat überführen. Die genannten Verbindungen sind zum Teil neu, sie sind zur Bekämpfung maligner Tumore einsetzbar und wirken dabei als Alkylantien.

Die folgenden Beispiele erläutern die Erfindung.

## Beispiele

### 1. N.N'-Diglycidyl-N''-(3-chlor-hydroxy-propyl)-urazol

Zu der Lösung von 26,9 g (0,1 Mol) 1,2,4-Triglycidylurazol (TGU) in 700 ml 10 %iger wäßriger Natriumchloridlösung wurden bei 40 °C so langsam insgesamt 36,5 g 10 %ige Salzsäure getropft, daß der pH-Wert bei 5 konstant gehalten werden konnte. Die Zugabe war nach ca. 70 Min. beendet. Danach wurde viermal mit je 250 ml Methylenchlorid extrahiert und das Methylenchlorid abdestilliert.

Nach dem Trocknen im Ölpumpenvakuum wurden 24,2 g (79 % d. Th.) DC-Reines N,N'-Diglycidyl-N''-(3-chlor-2-hydroxy-propyl)-urazol vom Brechungsindex $n_D^{20}$ : 1,511 5 erhalten.

Epoxid-O :

ber. 10,5 %
gef. 10,6 %

Analog wurden erhalten :

### 2. N, N'-Diglycidyl-N''-(3-brom-2-hydroxy-propyl)-urazol

HBr-Zutropfzeit : 65 Min.
$n_D^{20}$ : 1,518 3, Epoxid-O :

ber. 9,2 %,
gef. 8,8 %.

### 3. N,N'-Bis-(3-brom-2-hydroxy-propyl)-N''-glycidylurazol (neu)

HBr-Zutropfzeit : 150 Min.

$n_D^{20}$ : 1,534 2, Epoxid-O :

ber. 3,7 %,
gef. 3,8 %.

### 4. N,N'-Diglycidyl-N''-(3-iod-2-hydroxy-propyl)-urazol (neu)

In der Lösung von 13,5 g (0,05 Mol) TGU und 7,5 g (0,05 Mol) Natriumiodid in 300 ml Wasser wurden unter Rühren bei 40 °C 6,4 g (0,05 Mol) 10 % Jodwasserstoffsäure so zugetropft, daß der pH-Wert bei ca. 5 blieb. Nach ca. 13 Min. war die Zugabe beendet. Nach dem Aufarbeiten analog obiger Vorschrift wurden 18,5 g (94 % d. Th.) DC-reines, farbloses N,N'-Diglycidyl-N''-(3-iod-2-hydroxy-propyl)-urazol vom Berechnungsindex $n_D^{20}$ : 1,545 0 erhalten.

Epox-O :

ber. 8,1 %,
gef. 7,8 %.

### 5. Diglycidyl-(3-chlor-2-hydroxy-propyl)-isocyanurat (vergl. D 6068 P 30 37 094.6)

Zu der Lösung von 7,42 g (0,025 Mol) a-Triglycidylisocyanurat und 1,45 g (0,025 Mol) Natriumchlorid in 200 ml Wasser wurden 9 g 10 %ige Salzsäure so zugetropft, daß der pH-Wert zwischen 6 und 8 blieb. Nach beendeter Salzsäurezugabe (65 Min.) wurde wie vorher aufgearbeitet. Es wurden 6,4 g (77 % d. Th.) Diglycidyl-(3-chlor-2-hydroxy-propyl-isocyanurat als DC-reines (mit einer autentischen Probe identisch) farbloses viskoses Öl erhalten.

Epoxid-O :

ber 9,6 %,
gef. 9,0 %.

Die nach Beispiel 1-5 hergestellten Verbindungen erwiesen sich dünnschicht-chromatographisch als einheitlich. (Laufmittel Methylenchlorid : Essigester : Methanol 3 : 2 : 1, feste Phase Kieselgel 60 (E. Merck Darmstadt).

6. 1,2-Diglycidyl-4-(3-fluor-2-hydroxy-propyl)-urazol

Die Lösung von 1,5 g (5 mMol) 1,2-Diglycidyl-4-(3-chlor-2-hydroxy-propyl)-urazol in 100 ml Aceton wurde mit 2,1 g (50 mMol) Natriumfluorid versetzt und die Mischung 6 h zum Sieden erhitzt. Nach Abdestillieren des Lösungsmittels, Aufnehmen des Rückstandes in 50 ml Methylenchlorid, Filtrieren, Abdestillieren des Methylenchlorids und Trocknen bei 40 °C im Ölpumpenvakuum wurden 1,5 g (100 %) 1,2-Diglycidyl-4-(3-fluor-2-hydroxy-propyl)-urazol vom Brechungsindex $n_D^{20}$ :1,495 5 (farbl. Flüssigkeit) erhalten.

| | | |
|---|---|---|
| Epoxid-O | 10,9 gef. | 11,1 ber. |
| F | 6,0 gef. | 6,6 ber. |

Antitumorscreening (Leukämie der Ratte)

| Dosis (mg/kg) | 240 | 120 | 60 | 30 |
|---|---|---|---|---|
| T/C (%) | tox. | 272 | 202 | 182 |

Die T/C-Werte geben die prozentuale Verlängerung der Lebenszeit behandelter Tiere an.

7. Monoadditionsprodukt von Dikaliumhydrogenphosphat an 1,2,4-Triglycidyl-urazol (1 : 1 — Addukt)

26,9 g (0,1 Mol) 1,2,4-Triglycidyl-urazol (TGU) und 17,4 g (0,1 Mol) Dikaliumhydrogenphosphat wurden in 240 ml Wasser gelöst und auf 60 °C erwärmt. Innerhalb von drei Stunden wurden 26 ml 10 %iger Phosphorsäure zugetropft, wobei der pH-Wert bei 8,0 (plus/minus 0,5) gehalten wurde. Am Ende der Reaktionszeit wurde auf 20 °C abgekühlt und mit Methylenchlorid extrahiert, um nicht umgesetztes TGU zu entfernen. Die wäßrige Lösung wurde sodann gefriergetrocknet. Es wurden 47,7 g des 1 : 1 — Adduktes mit einem Restwassergehalt von 4 % erhalten.

| | |
|---|---|
| Freies Phosphat als P : | 2 % |
| Epoxid-Sauerstoff : | gefunden : 6,3 % |
| | berechnet : 6,6 % |

8. Die nachfolgenden Versuche wurden durchgeführt nach Testvorschriften des National Cancer Institute Bethesda, Maryland 200014, veröffentlicht in « Cancer Chemotherapy Reports » Part. 3, September 1972, Vol. 3, Nr. 2. Als Wirksubstanz wurden Addukte verschiedener Mineralsäuren an Triglycidylisocyanurat (TGU) verwendet. Die Substanzen wurden als wäßrige, 1 %ige Injektionslösungen unmittelbar vor der Applikation frisch hergestellt.

Bei Mäusen wurde gemäß Protokoll 1200 (S. 91c) die Tumorat P 388 (Leukämie) i. p. mit $10^6$ Zellen/Maus gesetzt. Die mittlere Überlebensdauer der unbehandelten Tiere wird bestimmt.

In weiteren Versuchsgruppen wird entsprechend vorbehandelten Tieren der Wirkstoff appliziert. Es wird eine signifikante Verlängerung der Lebensdauer der behandelten Testtiere gegenüber der mittleren Überlebensdauer der nicht mit dem Wirkstoff behandelten Tiere erzielt. Die Verlängerungsrate T/C in Abhängigkeit von der Dosierung des Wirkstoffes ist in der folgenden Tabelle zusammengestellt :

Tabelle zu Beispiel 8

Gemessene T/C — Werte

| Umsetzungsprodukt von 1 mol TGU mit: | verabreichte Dosis mg/kg | | |
|---|---|---|---|
| | 50 | 120 | 240 |
| 1 mol HCl | 283 | toxisch | toxisch |
| 1 mol HBr | 238 | 93 | toxisch |
| 2 mol HBr | 117 | 130 | 228 |
| 1 mol HJ | 155 | 200 | 285 |
| 1 mol NaH$_2$PO$_4$ | 113 | 137 | 141 |

**Patentansprüche**

1. Verfahren zur Herstellung definierter partieller Umsetzungsprodukte mehrfunktioneller N-Glycidylverbindungen mit Halogenwasserstoffsäuren, Pseudohalogenwasserstoffstäuren oder Säuren des Phosphors (unter Addition der Säuren an nur einen Teil der insgesamt vorliegenden Epoxidgruppen) dadurch gekennzeichnet, daß die mehrfunktionellen N-Glycidylverbindungen mit den Säuren und ihren Alkalimetallsalzen in wäßrigem Medium bei Temperaturen zwischen 0 und 60 °C im pH-Bereich zwischen 2 und 10 und dabei innerhalb einer Bandbreite von höchstens 2 Einheiten der pH-Skala bei einem Ansatzverhältnis von 1-20 mol Säure und ihrem Alkalimetallsalz pro mol umzusetzender Epoxidgruppe zur Reaktion gebracht werden, bis die vorbestimmte Menge an Epoxidgruppen abreagiert ist.

2. Verfahren nach Anspruch 1, dadurch gekennzeichnet, daß man zur Einstellung und/oder zur Konstanthaltung des pH-Werts die Säuren in dem Ausmaß dem Reaktionsgemisch zusetzt, wie sie durch die Reaktion verbraucht werden.

3. Verfahren nach Ansprüchen 1 und 2, dadurch gekennzeichnet, daß man die zu addierende Säure und vorzugsweise einen Überschuß der zu addierenden Säure in Form eines ihrer Alkalimetallsalze im wäßrigen Reaktionsmedium vorlegt und dann durch bevorzugt kontinuierliche Zugabe der freien Säure den pH-Wert regelt.

4. Verfahren nach Anspruch 3, dadurch gekennzeichnet, daß man dem Alkalimetallsalz enthaltenden wäßrigen Reaktionsgemisch Fremdsäuren zusetzt, die unter den Reaktionsbedingungen mit Epoxidgruppen vergleichsweise schwerer reagieren, und den pH-Wert mittels des Fremdsäurezusatzes regelt.

5. Verfahren nach Anspruch 4, dadurch gekennzeichnet, daß man als Fremdsäure Schwefelsäure oder organische Sulfonsäuren einsetzt.

6. Verfahren nach Ansprüchen 1 bis 5, dadurch gekennzeichnet, daß man in wäßriger Lösung praktisch neutral reagierende Alkalimetallsalze der zu addierenden Säuren einsetzt.

7. Verfahren nach Ansprüchen 1 bis 6, dadurch gekennzeichnet, daß man zunächst die Ringöffnung mit Chlorwasserstoff, Bromwasserstoff, Jodwasserstoff und/oder ihren Alkalimetallsalzen durchführt und anschließend in Gegenwart von Alkalimetallfluoriden durch Halogenaustausch die entsprechenden Fluorverbindungen herstellt.

8. Verfahren nach Ansprüchen 1 bis 7, dadurch gekennzeichnet, daß als mehrfunktionelle N-Glycidylverbindungen cyclische Verbindungen mit mehr als einer, bevorzugt ringständigen, —NR—CO-Gruppen verwendet werden, wobei R den Glycidylrest bedeutet.

9. Verfahren nach Ansprüchen 1 bis 8, dadurch gekennzeichnet, daß N-Glycidylverbindungen der Funktionalität 3 und höher zu Produkten mit 2 unumgesetzten Epoxidgruppen pro Molekül umgesetzt werden.

10. Verfahren nach den Ansprüchen 1 bis 9, dadurch gekennzeichnet, daß bei Temperaturen zwischen 20 und 40 °C gearbeitet wird.

11. Verfahren nach Ansprüchen 1 bis 10, dadurch gekennzeichnet, daß der pH des Reaktionsgemisches innerhalb einer Bandbreite von höchstens 2 Einheiten zwischen 3 und 8, vorzugsweise zwischen 4 und 7 konstant gehalten wird.

12. Verfahren nach den Ansprüchen 1 bis 11, dadurch gekennzeichnet, daß zur Erhöhung der Löslichkeit der N-Glycidylverbindungen in Wasser unter Reaktionsbedingungen inerte Lösungsmittel, Solubilisatoren und/oder Tenside zugesetzt werden.

**Claims**

1. A process for the production of defined partial trans-esterification products of polyfunctional N-glycidyl compounds with hydrohalic acids, pseudohydrohalic acids or acids of phosphorus (with addition of the acids to only some of the epoxide groups present), characterized in that the polyfunctional N-glycidyl compounds are reacted with the acids and their alkali metal salts in aqueous medium at temperatures of from 0 to 60 °C, at a pH value of from 2 to 10 and within a range of variation of at most 2 units of the pH scale and with a ratio of 1 to 20 moles acid and its alkali metal salt per mole epoxide group to be reacted, until the predetermined quantity of epoxide groups has reacted off.

2. A process as claimed in Claim 1, characterized in that, to adjust the pH value and/or to keep it constant, the acids are added to the reaction mixture at the rate at which they are consumed by the reaction.

3. A process as claimed in Claims 1 and 2, characterized in that the acid to be added and preferably an excess of the acid to be added in the form of one of its alkali metal salts is initially introduced into the reaction medium, after which the pH value is regulated by preferably continuous addition of the free acid.

4. A process as claimed in Claim 3, characterized in that foreign acids which react comparatively poorly with epoxide groups under the reaction conditions are added to the aqueous reaction mixture containing alkali metal salt and the pH value is regulated by addition of the foreign acid.

5. A process as claimed in Claim 4, characterized in that sulfuric acid or organic sulfonic acids are added as the foreign acid.

6. A process as claimed in Claims 1 to 5, characterized in that alkali metal salts of the acids to be added which show a substantially neutral reaction in aqueous solution are used.

7. A process as claimed in Claims 1 to 6, characterized in that the ring opening reaction is initially carried out with hydrogen chloride, hydrogen bromide, hydrogen iodide and/or alkali metal salts thereof, after which the corresponding fluorine compounds are prepared by halogen exchange in the presence of alkali metal fluorides.

8. A process as claimed in Claims 1 to 7, characterized in that cyclic compounds containing more than one —NR—CO-group (R = the glycidyl radical), preferably in the ring, are used as the polyfunctional N-glycidyl compounds.

9. A process as claimed in Claims 1 to 8, characterized in that N-glycidyl compounds having a functionality of 3 and higher are reacted to form products containing 2 unreacted epoxide groups per molecule.

10. A process as claimed in Claims 1 to 9, characterized in that it is carried out at temperatures of from 20 to 40 °C.

11. A process as claimed in Claims 1 to 10, characterized in that the pH of the reaction mixture is kept constant between 3 and 8 and preferably between 4 and 7 within a range of variation of at most 2 units.

12. A process as claimed in Claims 1 to 11, characterized in that solvents, solubilizers and/or surfactants which are inert under the reaction conditions are added to increase the solubility of the N-glycidyl compounds in water.

**Revendications**

1. Procédé pour la préparation de produits définis de réaction partielle de composés N-glycidyliques polyfonctionnels avec des hydracides halogénés, des hydracides pseudohalogénés ou des acides du phosphore (par addition des acides à une partie seulement de l'ensemble des groupes époxy existants), caractérisé en ce que l'on met en réaction les composés N-glycidyliques polyfonctionnels avec les acides et leurs sels de métaux alcalins, en milieu aqueux, à des températures comprises entre 0 et 60 °C, dans l'intervalle de pH situé entre 2 et 10, et dans cet intervalle, à l'intérieur d'une plage de 2 unités au plus de l'échelle des pH, en une proportion initiale de 1-20 moles d'acide et de son sel alcalin, par mole de groupe époxy à faire réagir, jusqu'à ce que la quantité préétablie de groupes époxy ait cessé de réagir.

2. Procédé selon la revendication 1, caractérisé en ce que, pour l'ajustement et/ou pour le maintien du pH, on ajoute les acides au mélange réactionnel suivant la quantité dans laquelle ils sont consommés par la réaction.

3. Procédé selon les revendications 1 et 2, caractérisé en ce que l'on dispose au préalable dans le milieu réactionnel aqueux l'acide à ajouter, et de préférence un excès de l'acide à ajouter, sous forme d'un de ses sels alcalins, et on effectue ensuite la régulation du pH par addition, de préférence continue, de l'acide libre.

4. Procédé selon la revendication 3, caractérisé en ce que l'on ajoute au mélange réactionnel aqueux contenant le sel de métal alcalin, des acides étrangers qui réagissent comparativement plus difficilement avec les groupes époxy dans les conditions réactionnelles, et on effectue la régulation du pH au moyen de l'addition d'acides étrangers.

5. Procédé selon la revendication 4, caractérisé en ce que l'on utilise en tant qu'acide étranger l'acide sulfurique ou des acides sulfoniques organiques.

6. Procédé selon l'une quelconque des revendications 1 à 5, caractérisé en ce que l'on utilise en solution aqueuse des sels alcalins à réaction pratiquement neutre des acides à ajouter.

7. Procédé selon l'une quelconque des revendications 1 à 6, caractérisé en ce que l'on effectue d'abord l'ouverture du cycle avec de l'acide chlorhydrique, bromhydrique, iodhydrique et/ou des sels alcalins de ces acides, et on prépare ensuite les composés fluorés correspondants, par échange d'halogènes en présence de fluorures de métaux alcalins.

8. Procédé selon l'une quelconque des revendications 1 à 7, caractérisé en ce que l'on utilise en tant que composés N-glycidyliques polyfonctionnels des composés cycliques comportant plus d'un groupe —NR—CO-, de préférence au noyau, R représentant le reste glycidyle.

9. Procédé selon l'une quelconque des revendications 1 à 8, caractérisé en ce que l'on transforme des composés N-glycidyliques à 3 fonctions et plus en produits comportant 2 groupes époxy inactifs par molécule.

10. Procédé selon l'une quelconque des revendications 1 à 9, caractérisé en ce que l'on opère à des températures comprises entre 20 et 40 °C.

11. Procédé selon l'une quelconque des revendications 1 à 10, caractérisé en ce que l'on maintient constant le pH du mélange réactionnel dans une plage de 2 unités ou plus, située entre 3 et 8, de préférence entre 4 et 7.

12. Procédé selon l'une quelconque des revendications 1 à 11, caractérisé en ce que l'on ajoute des solvants inertes, des solubilisants et/ou des agents tensio-actifs pour accroître la solubilité dans l'eau des composés N-glycidyliques dans les conditions réactionnelles.